# EUROPÄISCHE PATENTSCHRIFT

(11) **EP 3 393 568 B1**
(45) Veröffentlichungstag und Bekanntmachung des Hinweises auf die Patenterteilung: **21.08.2019**
(21) Anmeldenummer: 16820234.9
(22) Anmeldetag: 19.12.2016
(51) Int. Cl.: A61M 16/04, A61M 16/08

(54) **EINFÜHRHILFE**
INSERTION AID
ÉLÉMENT D'AIDE À L'INTRODUCTION

(30) Priorität: 23.12.2015 DE 102015122810
(43) Veröffentlichungstag der Anmeldung: 31.10.2018
(73) Patentinhaber: Tracoe Medical GmbH, 55268 Nieder-Olm (DE)
(72) Erfinder: SCHNELL, Ralf, 63500 Seligenstadt (DE)
(74) Vertreter: WSL Patentanwälte Partnerschaft mbB
(86) Internationale Anmeldenummer: PCT/EP2016/081786
(87) Internationale Veröffentlichungsnummer: WO 2017/108712

(56) Entgegenhaltungen:
- EP-A2- 1 044 701
- WO-A1-2006/087512
- DE-A1-102006 029 599
- DE-U1-202010 002 803
- US-A- 5 827 324

## Beschreibung

Die vorliegende Erfindung betrifft eine Vorrichtung zum Einführen einer Tracheostomiekanüle, umfassend ein Führungselement und einen Dilatator, wobei der Dilatator rohrförmig ist und das Führungselement zumindest abschnittsweise in den Dilatator einbringbar ist, wobei das Führungselement einen Schirm aufweist, der eine konische Form aufweist, mit einer Spitze und einer Schirmwand und einer Basis, wobei das Führungselement durch die Achse des Schirms und die Spitze verläuft und der Schirm im Bereich der Spitze mit dem Führungselement fest verbunden ist und wobei der Schirm einen ersten und einen zweiten Zustand aufweist und wobei der Schirm in dem ersten Zustand eine konische Form aufweist und in dem zweiten Zustand gegenüber dieser konischen Form verformt ist sowie eine Tracheostomiekanüle mit einer derartigen Einführhilfe.

Vorrichtungen der oben genannten Art, die für die Einführung einer Tracheostomiekanüle, insbesondere in ein noch aufzuweitendes Tracheostoma, geeignet sind, sind dem Fachmann bekannt. Die DE 10 2006 029 599 beschreibt eine derartige Einführhilfe, bei der an einem Führungselement ein Schirm angebracht ist, der einen in der Tracheostomiekanüle verlaufenden Dilatator im Bereich dessen Spitze, die an einem distalen Ende der Tracheostomiekanüle aus dieser herausragt, in eingebrachtem Zustand verdecken kann und einen stufenförmigen Kalibersprung am Übergang zwischen dem aus dem distalen Ende der Kanüle herausstehenden Dilatator und dem Außendurchmesser der Kanüle an ihrem distalen Ende überbrückt.

Insbesondere bei Kanülen mit großer Wandstärke am distalen Kanülenende kann ein derartiger abrupter Übergang für das Aufweiten eines Tracheostomas ein Problem darstellen, wenn das distale Ende der Kanüle sich beispielsweise an einer Trachealspange verkantet und damit eine Knorpelfraktur verursacht bzw. im Zuge des erhöhten Kraftaufwands für das Einbringen der Tracheostomiekanüle an die Rückwand der Trachea stößt und eine Verletzung derselben verursacht.

Die in der DE 10 2006 029 599 gezeigte Vorrichtung ermöglicht grundsätzlich ein sicheres Einbringen einer Tracheostomiekanüle in ein aufzuweitendes Tracheostoma. Die Vorrichtung erfordert jedoch eine sehr präzise Handhabung, bei der strengstens darauf geachtet werden muss, dass der auf den Dilatator aufgebrachte Schirm mit Führungselement beim Einführen der Kanüle fixiert bzw. festgehalten wird, um zu verhindern, dass das Führungselement mit Schirm durch die beim Aufweiten des Tracheostomas auf die Schirmspitze wirkende Kraft in Richtung proximales Ende der Tracheostomiekanüle geschoben wird. Wird dies nicht beachtet bzw. wird im Einzelfall die Tracheostomiekanüle während der Einführungsphase unvorsichtig zurückgezogen, besteht das Risiko, dass sich der Silikonschirm an dem Führungselement von der Spitze des Dilatators löst und somit nicht mehr das distale Ende der Tracheostomiekanüle bedeckt. Damit kann die in der DE 10 2006 029 599 gezeigte Vorrichtung nicht mehr ihren vorteilhaften Effekt ausüben und dies führt letztlich zu einer deutlich erschwerten Einführung der Tracheostomiekanüle in die Luftröhre, da ein atraumatischer Übergang zwischen Dilatator und Tracheostomiekanüle nicht mehr gewährleistet ist. Dies führt zu den oben beschriebenen Problemen, die bei einem nicht verdeckten Kalibersprung auftreten.

### Aufgabe

Vor dem Hintergrund dieses Stands der Technik liegt der vorliegenden Erfindung die Aufgabe zu Grunde, ein unbeabsichtigtes Lösen eines einen Kalibersprung verdeckenden Schirmes bei einer Einführhilfe für eine Tracheostomiekanüle zu verhindern.

Erfindungsgemäß wird diese Aufgabe gelöst durch eine Vorrichtung der eingangs genannten Art, wobei das Führungselement ein erstes Befestigungsmittel und der Dilatator ein korrespondierendes zweites Befestigungsmittel aufweist, wodurch das Führungselement mit dem Dilatator in einer Position durch Formschluss lösbar verbindbar ist, in der ein distales Ende des Dilatators mit einer Innenfläche des Schirms in Eingriff ist, wenn sich dieser in dem ersten Zustand befindet.

Eine derartige Ausgestaltung ermöglicht ein getrenntes Einbringen des Führungselements mit Schirm vom distalen Ende in die einzuführende Tracheostomiekanüle und des Dilatators vom proximalen Ende in die einzuführende Tracheostomiekanüle und anschließendes formschlüssiges Verbinden des Führungselements mit dem Dilatator in einer Position, in der der Schirm an dem Führungselement in seinem ersten Zustand mit einer Innenfläche mit dem distalen Ende des Dilatators in Eingriff steht und somit dessen distales Ende überdeckt. Damit ist der Schirm in der Lage, seine Funktion als Bestandteil der Vorrichtung auszuüben und den Kalibersprung zwischen Dilatator und Tracheostomiekanüle, die eingeführt werden soll, zu überdecken, wobei gleichzeitig ein unbeabsichtigtes Lösen verhindert wird.

Vor der Verwendung der erfindungsgemäßen Vorrichtung werden das Führungselement mit Schirm vom distalen Ende in die einzuführende Tracheostomiekanüle und der Dilatator vom proximalen Ende in die einzuführende Tracheostomiekanüle geschoben und anschließend Dilatator und Führungselement mit Hilfe der ersten und zweiten Befestigungsmittel miteinander verbunden. Zustäzlich wird dann der Schirm in seinen ersten Zustand gebracht, in dem der Dilatator mit der Innenfläche des Schirms in Eingriff steht. Ein Zusammenbau der erfindungsgemäßen Vorrichtung erfolgt somit innerhalb einer einzuführenden Tracheostomiekanüle.

Dies ermöglicht insbesondere auch die Verwendung der erfindungsgemäßen Vorrichtung für sehr enge und/oder stark gekrümmte Tracheostomiekanülen, in die eine derartige Vorrichtung zum Einführen sonst nicht einführbar wäre.

Das distale Ende des Dilatators ragt bei dem in eine Tracheostomiekanüle eingeführten Dilatator distal über die distale Öffnung der Tracheostomiekanüle hinaus. Die Form des hinausragenden Bereichs ist vorzugsweise im Wesentlichen konisch. Dementsprechend ist auch die Form der Innenfläche des Schirms im Wesentlichen konisch. Durch eine derartige Form findet eine besonders gute Kraftübertragung von dem Dilatator auf den Schirm statt, so dass mit Hilfe der Kombination aus einem derartigen Dilatator und Schirm eine Aufweitung des Tracheostomas und Einführen der Tracheostomiekanüle erleichtert wird.

Bei der perkutanen Dilatationstracheotomie, für welche die Vorrichtung vorgesehen ist, wird an der Stelle des geplanten Tracheostomas zunächst mit Hilfe einer Punktionsnadel ein Führungsdraht, auch Seldingerdraht genannt, in die Luftröhre eingebracht. Über den Führungsdraht wird ein Dilatator mit 14 French Außendurchmesser (14 French Dilatator) geführt und der vorhandene Punktionskanal erweitert, so dass anschließend ein Führungskatheter über den Führungsdraht in die Luftröhre eingebracht werden kann. Bei Verwendung der erfindungsgemäßen Vorrichtung handelt es sich dabei um das Führungselement. Mit Hilfe des Führungselements der Vorrichtung und dem Dilatator der Vorrichtung, wie sie hier beschrieben wird, erfolgt anschließend durch vorsichtiges Einschieben derselben in den Punktionskanal eine Dilatation des Punktionskanals, wobei der Führungsdraht als eine Art Leitschiene dient. Die Vorrichtung, die in eine Tracheostomiekanüle eingebracht ist und bei der der Schirm den Kalibersprung zwischen Dilatator und distalem Ende der Tracheostomiekanüle überdeckt, wird somit zur Aufweitung und schließlich Einführung der Tracheostomiekanüle in den geweiteten Punktionskanal geschoben. Anschließend wird der Dilatator zusammen mit dem damit verbundenen Führungselement mit Schirm in proximale Richtung aus der Tracheostomiekanüle herausgezogen. Es erfolgt daher mit Hilfe einer einzigen Vorrichtung das Aufweiten eines Punktionskanals zu einem Tracheostoma, in das gleichzeitig die Tracheostomiekanüle eingesetzt wird.

Die erfindungsgemäße Vorrichtung ist in gleicher Weise für einen Kanülenwechsel verwendbar, wobei durch das bereits bestehende Tracheostoma, das sich bei Entfernen der Tracheostomiekanüle zwar teilweise aber nicht vollständig zusammenzieht, keine erneute Punktion notwendig ist.

Anders als bei der herkömmlichen Tracheostomiekanüle nach Ciaglia ist es bei der erfindungsgemäßen Vorrichtung nicht zwingend erforderlich, das Tracheostoma zunächst mit einem oder mehreren separaten Dilatatoren zu erweitern, die dann wieder herausgezogen werden, um anschließend die Tracheostomiekanüle, ggf. mit einer Vorrichtung zum Einführen in das bereits geweitete Tracheostoma einzubringen.

Ungeachtet dessen kann selbstverständlich die erfindungsgemäße Vorrichtung - wie oben beschrieben - auch zum Einbringen einer Tracheostomiekanüle in ein bereits geweitetes Tracheostoma verwendet werden.

Bei der erfindungsgemäßen Einführhilfe ist es weiter vorgesehen, dass nach Einbringen der Tracheostomiekanüle in das Tracheostoma die in der Tracheostomiekanüle befindliche Vorrichtung mithilfe des Dilatators in Richtung proximales Ende aus der Tracheostomiekanüle herausgezogen wird.Durch die lösbare Verbindung des Führungselements mit dem Dilatator in verbundenem Zustand werden dabei sowohl Führungselement als auch Schirm zusammen mit dem Dilatator aus der Tracheostomiekanüle in Richtung proximales Ende zurückgezogen. Für dieses Rückziehen geht der Schirm von seinem ersten Zustand in seinen zweiten Zustand über. Bei dem Übergang des Schirmes von dem ersten in den zweiten Zustand kommen die Innenfläche des Schirms und das distale Ende des Dilatators zumindest teilweise außer Eingriff und der Schirm verformt sich derart, dass sein Außendurchmesser an jeder Stelle kleiner oder gleich dem Innendurchmesser der Tracheostomiekanüle ist, die mit der Vorrichtung eingebracht wird.

Eine formschlüssige Verbindung liefert eine besonders gute Absicherung gegenüber einem unbeabsichtigten Lösen. Dies schließt jedoch nicht aus, dass zu der formschlüssigen Verbindung zusätzlich auch ein Reibschluss zwischen den beiden Befestigungsmitteln vorliegen kann oder eine weitere lösbare Verbindung zwischen Führungselement und/oder Schirm einerseits und dem Dilatator andererseits.

Der Dilatator ist vorzugsweise nicht mit der Innenfläche des Schirms in Eingriff, wenn er in seinem zweiten Zustand ist. In einer Ausführungsform ist der Schirm in seinem zweiten Zustand zumindest teilweise oder vollständig umgestülpt im Vergleich zu dem ersten Zustand. Der Schirm ist in seinem zweiten Zustand so verformt, dass sein Außendurchmesser an jeder Stelle kleiner oder gleich dem Innendurchmesser der Tracheostomiekanüle ist, die mit der Vorrichtung eingebracht werden soll.

Die Begriffe "distal" und "proximal" wie sie hier verwendet werden beziehen sich auf den Standpunkt der die Tracheostomiekanüle mit der Vorrichtung einführenden Personen, d.h., dass das "distale" Ende das Ende oder den Bereich bezeichnet, der in den Patienten eingeführt wird und das "proximale" Ende das Ende oder den Bereich bezeichnet, der von dem Patienten weg nach außen weist. Beim Einführen der Einführhilfe wird somit zunächst ein distales Ende des Führungselements in den Patienten durch das aufzuweitende Tracheostoma eingeführt, wobei dies vorzugsweise über einen Führungsdraht erfolgt, der dann im Inneren des rohrförmigen Führungselements verläuft.

Das "Führungselement" bezeichnet im Sinne der Erfindung einen rohrförmigen Gegenstand. Bevorzugt weist das Lumen des rohrförmigen Führungselementes einen Innendurchmesser auf, der größer ist als der Außendurchmesser eines für eine Tracheostomie vorgesehenen Seldingerdrahtes, vorzugsweise größer als 1,3 mm. In einer Ausführungsform beträgt der Außendurchmesser des Seldingsdrahtes von 1 mm bis 1,3 mm, wobei bevorzugt der Innendurchmesser des Führungselementes zwischen 0,1 bis 0,5 mm größer ist, als der Außendurchmesser des Seidingerdrahtes.

Der Außendurchmesser des "Führungselements" ist zumindest an einem proximalen Abschnitt des Führungselements kleiner als der Innendurchmesser des Dilatators der Vorrichtung zum Einführen einer Tracheostomiekanüle und beträgt vorzugsweise von 2,7 mm bis 4 mm.

In einer Ausführungsform handelt es sich bei dem Führungselement um einen Führungskatheter, d.h. um ein schlauchförmiges Element mit im Wesentlichen konstanten Innen- und Außendurchmesser. In einer Ausführungsform ist der Außendurchmesser des Führungselements an verschiedenen Abschnitten größer oder kleiner als in anderen Abschnitten, vorzugsweise ist der Außendurchmesser in dem Bereich, in dem die Spitze des Schirms mit dem Führungselement verbunden ist, kleiner als in den übrigen Bereichen.

Bevorzugt verjüngt sich in einer Ausführungsform der Außendurchmesser des distalen Endes des Führungskatheters.

Der "Dilatator" bezeichnet ein Element, das zumindest an seinem distalen Ende und einem daran angrenzenden Abschnitt röhrenförmig ist, mit einem Außendurchmesser, der kleiner ist als der Innendurchmesser einer mit der Vorrichtung einzuführenden Tracheostomiekanüle. Der Dilatator weist weiter einen Schaft auf, der ebenfalls einen Außendurchmesser hat, der kleiner ist als der Innendurchmesser der einzuführenden Tracheostomiekanüle, und einen Griff, wobei der Griff zumindest an einem Abschnitt einen Außendurchmesser aufweist, der größer ist als der Innendruchmesser der Tracheostomiekanüle, so dass der Griff nicht in die Tracheostomiekanüle einführbar ist. Zumindest das distale Ende und der daran angrenzenden Abschnitt, weisen ein Lumen auf, mit einem Innendurchmesser, der größer oder gleich dem Außendurchmesser des Führungselementes ist, vorzugsweise beträgt der Innendurchmesser von 2,7 mm bis 4 mm. Dadurch ist das Führungselement zumindest in diesem Bereich in den Dilatator einschiebbar. Vorzugsweise ist der Außendurchmesser des Dilatators zumindest in dem an das distale Ende angrenzenden Abschnitt von 0,2 mm bis 4 mm, vorzugsweise von 1 mm bis 1,5 mm, kleiner als der Innendurchmesser der mit der Vorrichtung einzuführenden Tracheostomiekanüle. Vorzugsweise weist der an das distale Ende angrenzende Abschnitt eine axiale Länge von 2 mm bis 4 cm, bevorzugt 5 mm bis 2 cm auf.

In einer Ausführungsform hat der Dilatator die Form eines herkömmlichen Dilatators, d.h. es handelt sich um ein vollständig rohrförmiges Element, das zumindest abschnittsweise gebogen ist, mit einem vorzugsweise konischen distalen Ende und einem proximalen Griffbereich, wobei der Griffbereich zumindest an einem Abschnitt einen Außendurchmesser aufweist, der größer ist als der Innendurchmesser der mit der Vorrichtung einzuführenden Tracheostomiekanüle. Eine derartige Ausführungsform ist zur Verwendung mit einem Führungselement geeignet, das als Führungskatheter ausgestaltet ist, wobei der Führungskatheter länger ist, als die axiale Länge einer mit der Vorrichtung einzuführenden Tracheostomiekanüle.

In einer Ausführungsform schließt sich an den an das distale Ende angrenzenden Abschnitt als Schaft ein flexibler stab- oder röhrenförmiger Bereich an, dessen Außendurchmesser weniger als die Hälfte des Innendurchmessers der mit der Vorrichtung einzuführenden Tracheostomiekanüle beträgt. Bei einer Ausführungsform mit stabförmigem Schaft verläuft ein durch den Dilatator bei der Einbringung hindurchzuführender Seldingerdraht im Gegensatz zu anderen Ausführungsformen mit vollständig rohrförmigem Dilatator nicht durch den Schaft des Dilatators sondern entlang des Schafts.

Der Schirm befindet sich an dem Führungselement vorzugsweise näher an dem distalen Ende des Führungselements als an dem proximalen Ende des Führungselements und seine Spitze weist in Richtung des distalen Endes des Führungselements.

Die Formulierung, dass "das Führungselement ein erstes Befestigungsmittel [...] aufweist", wie sie hierin verwendet wird, umfasst, dass sich das erste Befestigungsmittel unmittelbar an dem Führungselement oder an dem mit dem Führungselement verbundenen Schirm befindet.

Der Begriff "konische Form" wie er hier verwendet wird, bezieht sich darauf, dass der im Wesentlichen aus einer Spitze und einer Schirmwand bestehende Schirm wenigstens in seiner ersten Position eine Schirmwand mit einem von der Spitze zu der Basis hin ohne sprunghafte Aufweitung zunehmenden Außendurchmesser hat, wobei die Kontur des Konus auch konkav oder konvex gewölbt sein kann. Die Schirmwand weist eine Außenfläche und die Innenfläche des Schirms auf, wobei die konische Form im Wesentlichen durch die Außenfläche der Schirmwand gebildet wird. Die Schirmwand weist zumindest in dem ersten Zustand vorzugsweise im Wesentlichen die Form eines Hohlkegels auf.

Der Begriff "Basis" des Schirmes bezeichnet die Ebene des Schirmes, insbesondere der Schirmwand, senkrecht zu seiner Achse, die den größten Durchmesser hat.

Vorzugsweise befindet sich das distale Ende der Spitze des Schirmes in einem Abstand von 1 mm bis 8 cm, bevorzugt 3 mm bis 5 cm, vom distalen Ende des Führungselements. In einer anderen Ausführungsform befindet sich die Spitze des Schirmes am distalen Ende des Führungselements und dieses ragt distal nicht über die Spitze des Schirmes hinaus.

Vorzugsweise beträgt die axiale Länge des Schirms zwischen 1 cm und 7 cm, besonders bevorzugt zwischen 1,5 cm und 4 cm.

Bevorzugt ist auch, dass der Konuswinkel zwischen dem Schirm und der Längsachse des Führungselements zwischen 2° und 20°, bevorzugt zwischen 5° und 11° beträgt. Es ist bevorzugt, wenn der Schirm in dem ersten Zustand im Wesentlichen die Form eines Hohlkegels hat und innen und außen konisch geformt ist.

Bevorzugt ist ebenfalls, dass der Schirm so ausgearbeitet ist, dass er sich teilweise oder ganz umstülpt, um von dem ersten in den zweiten Zustand zu kommen.

In einer Ausführungsform weist die Basis des Schirmes einen hohlzylinderförmigen Ansatz auf, mit einem Außendurchmesser, der dem Innendurchmesser einer einzuführenden Tracheostomiekanüle angepasst ist und diesen um maximal 10%, bevorzugt maximal 5%, bezogen auf den Innendurchmesser der einzuführenden Tracheostomiekanüle, über- oder unterschreitet, und mit einer Wandstärke, die zwischen 0,5 mm und 2 mm, bevorzugt zwischen 0,5 mm und 1 mm beträgt. Vorteilhaft ist es, wenn der Schirm eine maximale Wandstärke an der Basis von höchstens 2,5 mm, bevorzugt höchstens 1,8 mm und wenigstens 0,4 mm, bevorzugt wenigstens 0,5 mm, aufweist.

In einer Ausführungsform besteht der Schirm aus oder umfasst Silikon oder thermoplastische Elastomere, einschließlich thermoplastische Elastomere auf Olefinbasis (TPO), thermoplaste Elastomere auf Urethanbasis (TPU), Thermoplastische Polyesterelastomere (TPC), einschließlich Copolyester, Stryol Blockcopolymere (TPS), einschließlich SBS, SEBS; SEPS, SEEPS und MBS, oder thermoplastische Copolyamide (TPA).

Vorzugsweise weist der Schirm an seiner Basis in dem ersten Zustand einen Außendurchmesser auf, der größer ist als der Innendurchmesser der mit der Vorrichtung einzuführenden Tracheostomiekanüle an ihrem distalen Ende, bevorzugt dem Außendurchmesser einer mit der Vorrichtung einzuführenden Tracheostomiekanüle an ihrem distalen Ende entspricht, und hat in seinem zweiten Zustand durch Verformung einen kleineren Außendurchmesser als den Innendurchmesser der Tracheostomiekanüle, sodass er durch diese durchziehbar ist. Die Verformung des Schirms beim Übergang zwischen erstem und zweitem Zustand erfolgt vorzugsweise durch vollständiges oder teilweises Umstülpen, insbesondere beim Herausziehen des Dilatators aus der Tracheostomiekanüle. Der Begriff "dem Außendurchmesser einer mit der Vorrichtung einzuführenden Tracheostomiekanüle an ihrem distalen Ende entspricht" in Bezug auf den Außendurchmesser der Basis des Schirmes umfasst im Sinne der Erfindung auch Schirme mit einem Außendurchmesser an der Basis, der aufgrund von Toleranzabweichungen bis zu 0,5 mm größer oder kleiner als der Außendurchmesser der Tracheostomiekanüle an ihrem distalen Ende ist.

Gleichermaßen weist der Dilatator einen Außendurchmesser auf, der kleiner ist als der Innendurchmesser der mit der Vorrichtung einzuführenden Tracheostomiekanüle. Der rohrförmige Dilatator weist weiter vorzugsweise einen Innendurchmesser auf, der zumindest in einem distalen Abschnitt des Dilatators größer ist als der Außendurchmesser des Führungselements und vorzugsweise kleiner ist als der jeweils größte Außendurchmesser des Schirmes in dem ersten und/oder zweiten Zustand, sodass das Führungselement mit Schirm nicht durch den Dilatator hindurchführbar ist.

Es versteht sich, dass die Außenkontur des Dilatators vorzugsweise zumindest in einem Abschnitt im Wesentlichen die Innenkontur der mit der Vorrichtung einzuführenden Tracheostomiekanüle aufweist und insbesondere deren Krümmung. Vorzugsweise weist der Dilatator an seinem proximalen Ende einen Griff auf.

Bevorzugt ist auch, dass sich das zweite Befestigungsmittel an dem Dilatator an dem distalen Ende befindet und sich das erste Befestigungsmittel an dem Führungselement in einem Bereich befindet, der in unverbundenem Zustand der Befestigungsmittel in dem ersten Zustand des Schirms von dem Schirm bedeckt ist und in dem zweiten Zustand des Schirms zumindest teilweise sichtbar ist.

Eine derartige Anordnung ermöglicht es, nach dem Einbringen des Dilatators in die Tracheostomiekanüle, wobei dieses Einbringen vorzugsweise vom proximalen Ende her erfolgt, aus distaler Richtung das Führungselement soweit durch den Dilatator hindurch zu führen, bis sich der Schirm im Bereich des distalen, ersten Endes des Dilatators befindet. Durch manuelles Verbringen des Schirmes in den zweiten Zustand ist es so möglich, das erste Befestigungsmittel mit dem zweiten Befestigungsmittel zu verbinden und anschließend den Schirm wieder in den ersten Zustand zu verbringen, sodass beide Befestigungsmittel von dem Schirm zumindest weitestgehend bedeckt sind.

In einer Ausführungsform befindet sich das erste Befestigungsmittel außen an dem Führungselement oder an einer um das Führungselement angeordneten Manschette, die an dessen Außenwand anliegt und Bestandteil des Schirms sein kann.

In einer Ausführungsform bilden das erste Befestigungsmittel und das zweite Befestigungsmittel einen Drehverschluss, vorzugsweise einen Gewindeverschluss oder einen Bajonettverschluss. Diesen Verschlüssen ist gemein, dass ein Verbinden und Lösen der Verbindung durch eine Drehbewegung erfolgt.

In einer Ausführungsform befindet sich bei dem Gewindeverschluss ein Außengewinde am äußeren Umfang des Führungselements und ein Innengewinde am inneren Umfang des rohrförmigen Dilatators.

In einer Ausführungsform befindet sich bei einem Bajonettverschluss eine Nase an dem äußeren Umfang des Führungselements, während die für das Einrasten des Bajonettverschlusses notwendige Vertiefung sich an dem inneren Umfang des rohrförmigen Dilatators befindet.

In einer Ausführungsform ist das erste Befestigungsmittel mit dem Führungselement und/oder das zweite Befestigungsmittel mit dem Dilatator einstückig ausgebildet.

In einer Ausführungsform bilden das erste Befestigungsmittel und das zweite Befestigungsmittel eine Rastverbindung. Bei einer derartigen Rastverbindung weist das erste Befestigungsmittel oder das zweite Befestigungsmittel wenigstens eine Rastnase auf, die vorzugsweise mit federnder Vorspannung in eine entsprechende Vertiefung an dem jeweils anderen Befestigungsmittel einrastet.

In bestimmten Ausführungsformen sind auch Kombinationen unterschiedlicher lösbarer Verbindungen zwischen Führungselement und/oder Schirm einerseits und dem Dilatator andererseits denkbar und im Sinne der Erfindung.

In einer Ausführungsform weist der Silikonschirm an seiner Innenfläche eine Rillenstruktur auf und der Dilatator an dem distalen Ende eine korrespondierende Rillenstruktur, wobei die Rillen der Rillenstruktur sich vorzugsweise in Längsrichtung erstrecken. Die Rillenstruktur bildet eine weitere Absicherung gegenüber einem unbeabsichtigten Lösen des Schirms von dem Dilatator.

In einer Ausführungsform wird eine sich in Längsrichtung erstreckende Rillenstruktur mit einem Drehverschluss kombiniert. Bei einer derartigen Kombination verhindert die Rillenstruktur zusätzlich eine Lockerung der Drehverbindung durch Verdrehen von Dilatator und Schirm gegeneinander, da sie eine im Hinblick auf die Drehbewegung formschlüssige Verbindung bildet.

In einer Ausführungsform liegt der Schirm mit seiner Innenfläche an dem distalen Ende des Dilatators in verbundenem Zustand der Befestigungsmittel und in dem ersten Zustand des Schirms mit einer elastischen Vorspannung an. Dies stellt eine weitere Sicherungsfunktion der Befestigung dar, die ein unbeabsichtigtes Lösen beispielsweise einer durch Formschluss geschaffenen Verbindung durch einen Reibschluss zusätzlich verhindert.

Die oben genannte Aufgabe wird auch gelöst durch eine Tracheostomiekanüle mit einer oben beschriebenen Einführhilfe.

In einer Ausführungsform weist der Dilatator im Bereich seines proximalen Endes ein drittes Befestigungsmittel auf, mit dem der Dilatator mit dem proximalen Ende des Kanülenrohrs und/oder mit einem vierten Befestigungsmittel am proximalen Ende des Kanülenrohrs lösbar verbindbar ist. Mit Hilfe dieses dritten Befestigungsmittels, mit dem zusätzlich zu der Fixierung des Führungselements an dem Dilatator auch der Dilatator an der Tracheostomiekanüle lösbar befestigt wird, wird ein Verschieben des Dilatators in der Tracheostomiekanüle in Längsrichtung während des Einführens der Tracheostomiekanüle verhindert. Durch eine derartige Ausgestaltung wird eine zusätzliche Fehlerquelle ausgeschlossen und dem die Tracheostomiekanüle Einführenden weiter die Handhabung vereinfacht.

In einer Ausführungsform befindet sich das vierte Befestigungsmittel unmittelbar an dem Kanülenrohr der Tracheostomiekanüle.

Vorzugsweise umfasst das dritte Befestigungsmittel eine Überwurfmutter und/oder eine Klemme und/oder einen Konnektor. Es versteht sich, dass bei einer Überwurfmutter das proximale Ende des Kanülenrohres ein entsprechendes Gewinde aufweisen muss, um eine Befestigung zu gewährleisten.

Ein Konnektor wird insbesondere bei Tracheostomiekanülen verwendet, bei denen ein Konnektor als viertes Befestigungsmittel für die Befestigung eines Beatmungsschlauches am proximalen Ende des Kanülenrohrs vorgesehen ist. Das dritte Befestigungselement ist bei einer derartigen reibschlüssigen Verbindung so ausgestaltet, dass es von außen an dem sich in Richtung proximales Ende verjüngenden im Wesentlichen konischen Konnektor anliegt und zu dessen Form korrespondiert.

Weitere Vorteile, Merkmale und Anwendungsmöglichkeiten der vorliegenden Erfindung werden deutlich anhand der folgenden Beschreibungen bevorzugter Ausführungsformen und den dazugehörigen Figuren. Es versteht sich, dass die Figuren lediglich schematische Darstellungen sind und keinesfalls als maßstabs- oder winkelgetreue Wiedergaben verstanden werden sollen. Ungeachtet der verwendeten Schraffierungen bestehen die in den Figuren dargestellten Elemente der Ausführungsformen vorzugsweise aus Kunststoff. Es zeigen:
- Figur 1:: einen Ausschnitt eines Längsschnitts einer Ausführungsform einer erfindungsgemäßen Einführhilfe,
- Figur 2:: einen Ausschnitt eines Längsschnitts einer weiteren Ausführungsform einer erfindungsgemäßen Einführhilfe,
- Figur 3:: einen Ausschnitt eines Längsschnitts einer weiteren Ausführungsform einer erfindungsgemäßen Einführhilfe,
- Figur 4:: einen Längsschnitt durch eine erfindungsgemäße Einführhilfe, die sich in verbundenem Zustand innerhalb einer Tracheostomiekanüle befindet, und
- Figur 5:: einen Längsschnitt durch eine weitere erfindungsgemäße Einführhilfe, die sich in verbundenem Zustand innerhalb einer Tracheostomiekanüle befindet.

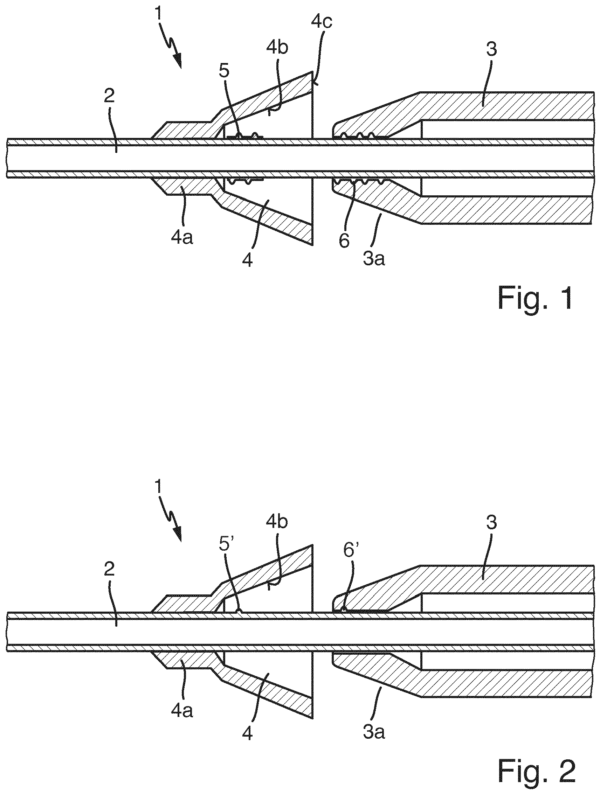

In Figur 1 ist eine erfindungsgemäße Vorrichtung 1 zum Einführen einer Tracheostomiekanüle im Querschnitt dargestellt, die ein Führungselement 2 sowie einen Dilatator 3 umfasst. An dem Führungselement 2 befindet sich ein Schirm 4, der im Bereich seiner Spitze 4a fest mit dem Führungselement verbunden ist. An dem Führungselement befindet sich weiter ein erstes Befestigungsmittel 5, bei dem es sich bei dieser Ausführungsform um ein Außengewinde handelt. In dem in Figur 1 dargestellten ersten Zustand des Schirmes befindet sich bei dieser Ausführungsform das erste Befestigungsmittel in einem Bereich, der vollständig von dem Schirm bedeckt ist. Am distalen Ende 3a des Dilatators 3 befindet sich ein zweites Befestigungsmittel 6, das bei dieser Ausführungsform ein Innengewinde ist, und zu dem ersten Befestigungsmittel 5 an dem Führungselement 2 korrespondiert.

Weiter weist der rohrförmige Dilatator ein Lumen auf, dessen Querschnitt an jeder Stelle zumindest etwas größer ist als der Außendurchmesser des Führungselementes, sodass das Führungselement, mit allen Bereichen, an denen sich kein Befestigungsmittel oder Schirm befindet, in den Dilatator einschiebbar ist.

Aus Figur 1 ist ebenfalls deutlich erkennbar, dass im verbundenen Zustand der ersten und zweiten Befestigungsmittel das distale Ende 3a des Dilatators mit der Innenfläche 4b des Schirmes, sofern der Schirm, wie in Figur 1 gezeigt, in seinem ersten Zustand ist, in Eingriff ist. Die konische Form des Schirmes wird somit durch den Dilatator in dem verbundenen Zustand und in dem ersten Zustand des Schirmes unterstützt und der Dilatator verleiht dem Schirm eine für die Ausweitung eines Tracheostomas erforderliche Stabilität.

Die in Figur 2 gezeigte Ausführungsform entspricht im Wesentlichen der in Figur 1 gezeigten. Die Einführhilfe 1 weist ein Führungselement 2 und einen Dilatator 3 auf. An dem Führungselement 2 befindet sich ein Schirm 4, wobei der Schirm mit seiner Spitze 4a fest mit dem Führungselement 2 verbunden ist. Weiter befindet sich an dem Führungselement 2 ein erstes Befestigungsmittel 5' und an dem Dilatator 3 ein zweites Befestigungsmittel 6', wobei das zweite Befestigungsmittel auch bei dieser Ausführungsform sich im Wesentlichen im distalen Bereich des Dilatators 3 befindet.

Bei dieser Ausführungsform wird durch das erste und zweite Befestigungsmittel 5', 6' ein Bajonettverschluss gebildet, wobei eine Befestigung durch Formschluss ebenfalls wie bei dem in Figur 1 gezeigten Drehverschluss durch Durchführen einer Drehbewegung zustande kommt.

Auch bei dieser Ausführungsform korrespondiert das distale Ende 3a des Dilatators 3 im Wesentlichen mit der Innenfläche 4b des Schirmes 4, sodass in einer Position, in der das Führungselement mit dem Dilatator verbunden ist, wenn sich der Schirm in dem ersten Zustand befindet, das distale Ende 3a des Dilatators 3 mit der Innenfläche 4b des Schirmes 4 in Eingriff ist.

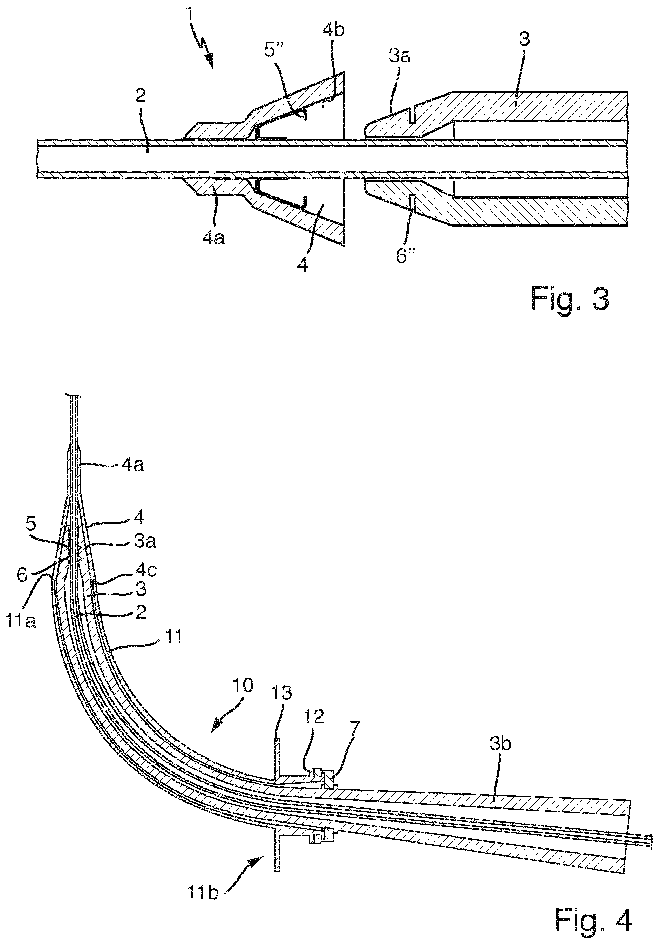

Figur 3 zeigt ebenfalls eine erfindungsgemäße Ausführungsform einer Vorrichtung 1 zur Einführung einer Tracheostomiekanüle, die ein Führungselement 2 und einen Dilatator 3 umfasst. An dem Führungselement 2 befindet sich ein Schirm 4, der mit seiner Spitze 4a fest mit dem Führungselement 2 verbunden ist. Das Führungselement weist weiter ein erstes Befestigungsmittel 5" auf, bei dem es sich um zwei vorgespannte Klammern handelt und der Dilatator weist ein zweites Befestigungsmittel 6" auf, bei dem es sich um zwei Vertiefungen handelt, mit denen die Klammern im verbundenen Zustand in Eingriff stehen. Die Klammern des ersten Befestigungsmittels 5" sind so vorgespannt, dass ein Lösen der Verbindung mit dem zweiten Befestigungsmittel 6" nur durch Überwinden der Vorspannung möglich ist.

Auch bei dieser Ausführungsform korrespondiert das distale Ende des Dilatators 3a im Wesentlichen mit der Innenfläche des Schirmes 4b, sodass im verbundenen Zustand, wenn der Schirm sich in seinem ersten Zustand befindet, das distale Ende des Dilatators mit der Innenfläche des Schirmes in Eingriff ist.

In Figur 4 ist eine Tracheostomiekanüle 10 mit einer erfindungsgemäßen Vorrichtung 1 dargestellt, wobei die Vorrichtung 1 im Wesentlichen der in Figur 1 gezeigten Ausführungsform entspricht, das bedeutet, dass das erste Befestigungsmittel 5 und das zweite Befestigungsmittel 6 eine Drehverbindung bilden, wobei das erste Befestigungsmittel ein an dem Führungselement 2 außen angebrachtes Außengewinde ist und das zweite Befestigungsmittel ein sich im Lumen des Dilatators befindliches Innengewinde ist. In verbundenem Zustand sind sowohl das erste Befestigungsmittel 5 und das zweite Befestigungsmittel 6 als auch das distale Ende 3a des Dilatators 3 von dem Schirm bedeckt, der sich an dem Führungselement 2 befindet. Das distale Ende des Dilatators 3a tritt dabei mit der Innenfläche 4b des Schirmes 4, der sich in seinem ersten Zustand befindet, in Eingriff. Das Führungselement 2 ist in dieser Ausführungsform als ein Führungskatheter ausgestaltet.

Die Tracheostomiekanüle 10 weist ein Kanülenrohr 11 auf, wobei in Figur 4 deutlich zu erkennen ist, dass die Basis 4c des Schirmes 4 das distale Ende 11a des Kanülenrohres bedeckt, wobei die Basis 4c des Schirmes 4 einen Außendurchmesser aufweist, der im Wesentlichen dem Außendurchmesser des Kanülenrohres 11 entspricht bzw. etwas größer ist. Durch diese Ausgestaltung wird der Kalibersprung zwischen dem distalen Ende 3a des Dilatators 3 und dem distalen Ende des Kanülenrohres 11a vollständig von dem Schirm bedeckt.

Bei der in Figur 4 gezeigten Ausführungsform befindet sich weiter an dem proximalen Ende 11b des Kanülenrohres 11 ein viertes Befestigungsmittel 12, das mit einem dritten Befestigungsmittel 7 an dem Dilatator 3 in der in Figur 4 gezeigten Position verbunden ist. Bei dem dritten Befestigungsmittel 7 handelt es sich bei dieser Ausführungsform um eine Überwurfmutter, die in eine entsprechende Vertiefung bzw. ein Gewinde des vierten Befestigungsmittels 12 eingreift. Der Griff des Dilatators 3b ist zusätzlich so gestaltet, dass er auch unabhängig von dem dritten Befestigungsmittel 7 nicht in das Kanülenrohr 11 der Tracheostomiekanüle 10 einschiebbar ist, da sein Außendurchmesser größer ist als der innendurchmesser der Tracheostomiekanüle 10.

An der Tracheostomiekanüle 10 ist lediglich schematisch ein Schild 13 angedeutet, mit dem mit Hilfe eines Kanülenbandes die Tracheostomiekanüle am Hals eines Patienten befestigbar ist. Die genaue Ausgestaltung des Schildes spielt jedoch für die vorliegende Erfindung keine Rolle, sodass dieses nur der Vollständigkeit halber rudimentär angedeutet ist.

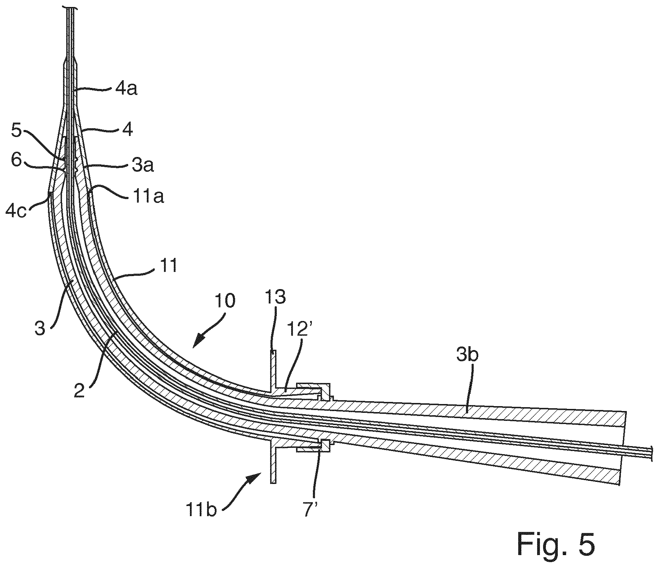

In Figur 5 ist ebenfalls eine Tracheostomiekanüle 10 mit einer erfindungsgemäßen Vorrichtung 1 dargestellt. Die in Figur 5 gezeigte Ausführungsform entspricht im Wesentlichen der in Figur 4 gezeigten, sodass auf die Ausführungen dort verwiesen wird.

Die Ausführungsform in Figur 5 unterscheidet sich jedoch in der Art des dritten Befestigungsmittels 7' und vierten Befestigungsmittels 12' von der in Figur 4 gezeigten. Bei den in Figur 5 gezeigten Befestigungen handelt es sich um eine reibschlüssige Verbindung. Dabei verjüngt sich das vierte Befestigungsmittel 12' an dem proximalen Ende 11b des Kanülenrohrs 11 in proximale Richtung und weist die Form eines üblichen 15 mm-Konnektors auf. Das dritte Befestigungsmittel 7' an dem Dilatator 3 weist eine zu der äu ßeren Oberfläche des vierten Befestigungsmittels 12' korrespondierende innere Oberfläche auf, wobei das dritte Befestigungsmittel 7'in verbundenem Zustand reibschlüssig an der Außenfläche des vierten Befestigungsmittels 12' anliegt.

### Bezugszeichenliste

- 1: Vorrichtung zum Einführen einer Tracheostomiekanüle
- 2: Führungselement
- 3: Dilatator
- 3a: distales Ende des Dilatators
- 3b: Griff des Dilatators
- 4: Schirm
- 4a: Spitze des Schirms
- 4b: Innenfläche des Schirms
- 4c: Basis des Schirms
- 5, 5', 5": erstes Befestigungsmittel
- 6, 6', 6": zweites Befestigungsmittel
- 7, 7`: drittes Befestigungsmittel
- 10: Tracheostomiekanüle
- 11: Kanülenrohr
- 11a: distales Ende des Kanülenrohrs
- 11b: proximales Ende des Kanülenrohrs
- 12, 12': viertes Befestigungsmittel
- 13: Schild

## Patentansprüche

1. Vorrichtung (1) zum Einführen einer Tracheostomiekanüle, umfassend ein Führungselement (2) und einen Dilatator (3), wobei der Dilatator (3) rohrförmig ist und das Führungselement (2) zumindest abschnittsweise in den Dilatator (3) einbringbar ist, wobei das Führungselement (2) einen Schirm (4) aufweist, mit einer Spitze (4a) und einer Schirmwand und einer Basis (4c), wobei das Führungselement (2) durch die Achse des Schirms (4) und die Spitze (4a) verläuft und der Schirm (4) im Bereich der Spitze (4a) mit dem Führungselement (2) fest verbunden ist und wobei der Schirm (4) einen ersten und einen zweiten Zustand aufweist und wobei der Schirm (4) in dem ersten Zustand eine konische Form aufweist und in dem zweiten Zustand gegenüber dieser konischen Form verformt ist, **dadurch gekennzeichnet, dass**
das Führungselement (2) ein erstes Befestigungsmittel (5, 5', 5") und der Dilatator (3) ein korrespondierendes zweites Befestigungsmittel (6, 6', 6") aufweist, wodurch das Führungselement (2) mit dem Dilatator (3) in einer Position durch Formschluss lösbar verbindbar ist, in der ein distales Ende des Dilatators (3) mit einer Innenfläche des Schirms (4b) in Eingriff ist, wenn sich dieser in dem ersten Zustand befindet.

2. Vorrichtung (1) gemäß Anspruch 1, **dadurch gekennzeichnet, dass** der Schirm (4) an seiner Basis (4c) in dem ersten Zustand einen Außendurchmesser aufweist, der größer ist als der Innendurchmesser der mit der Vorrichtung (1) einzuführenden Tracheostomiekanüle (10) an ihrem distalen Ende (11a), bevorzugt dem Außendurchmesser einer mit der Vorrichtung (1) einzuführenden Tracheostomiekanüle (10) an ihrem distalen Ende (11a) entspricht, und in seinem zweiten Zustand durch Verformung einen kleineren Außendurchmesser als den Innendurchmesser der mit der Vorrichtung (1) einzuführenden Tracheostomiekanüle (10).

3. Vorrichtung (1) gemäß einem der Ansprüche 1 oder 2, **dadurch gekennzeichnet, dass** sich das zweite Befestigungsmittel (6, 6', 6") an dem Dilatator (3) an dem distalen Ende (3a) befindet und sich das erste Befestigungsmittel (5, 5', 5") an dem Führungselement (2) in einem Bereich befindet, der in unverbundenem Zustand der Befestigungsmittel (5, 5', 5", 6, 6', 6") in dem ersten Zustand des Schirms (4) von dem Schirm (4) bedeckt ist und in dem zweiten Zustand des Schirms (4) zumindest teilweise sichtbar ist.

4. Vorrichtung (1) gemäß einem der vorangehenden Ansprüche, **dadurch gekennzeichnet, dass** das erste Befestigungsmittel (5, 5', 5") und das zweite Befestigungsmittel (6, 6', 6") einen Drehverschluss bilden, vorzugsweise einen Gewindeverschluss (5, 6) oder einen Bajonettverschluss (5', 6').

5. Vorrichtung (1) gemäß einem der Ansprüche 1 bis 3, **dadurch gekennzeichnet, dass** das erste Befestigungsmittel (5, 5', 5") und das zweite Befestigungsmittel (6, 6', 6") eine Rastverbindung (5", 6") bilden.

6. Vorrichtung (1) gemäß einem der vorangehenden Ansprüche, **dadurch gekennzeichnet, dass** der Schirm (4) aus Silikon hergestellt ist oder Silikon umfasst, wobei an seiner Innenfläche der Schirm (4) eine Rillenstruktur aufweist und der Dilatator (3) an dem distalen Ende (3a) eine korrespondierende Rillenstruktur, wobei die Rillen der Rillenstruktur sich vorzugsweise in Längsrichtung erstrecken.

7. Vorrichtung (1) gemäß einem der vorangehenden Ansprüche, **dadurch gekennzeichnet, dass** der Schirm (4) mit seiner Innenfläche (4b) an dem distalen Ende (3a) des Dilatators (3)in verbundenem Zustand der Befestigungsmittel (5, 5', 5", 6, 6', 6") und in dem ersten Zustand des Schirms (4) mit einer elastischen Vorspannung anliegt.

8. Set mit einer Tracheostomiekanüle (10) und einer Vorrichtung (1) gemäß einem der Ansprüche 1 bis 7.

9. Set gemäß Anspruch 8, **dadurch gekennzeichnet, dass** die Tracheostomiekanüle (10) ein Kanülenrohr (11) mit einem proximalen Ende (11b) und der Dilatator (3) im Bereich seines proximalen Endes ein drittes Befestigungsmittel (7, 7') aufweist, mit dem der Dilatator (3) mit dem proximalen Ende (11b) des Kanülenrohrs (11) und/oder mit einem vierten Befestigungsmittel (12, 12') am proximalen Ende (11b) des Kanülenrohrs (11) lösbar verbindbar ist.

10. Set gemäß Anspruch 9, **dadurch gekennzeichnet, dass** das dritte Befestigungsmittel (12, 12') eine Überwurfmutter oder eine Klemme oder einen Konnektor umfasst.

## Claims

1. Device (1) for inserting a tracheostomy tube, comprising a guide element (2) and a dilator (3), wherein the dilator (3) is tubular and the guide element (2) can be introduced into the dilator (3) at least in sections, wherein the guide element (2) has a shield (4), with a tip (4a) and a shield wall and a base (4c), wherein the guide element (2) passes through the axis of the shield (4) and the tip (4a), and the shield (4) in the area of the tip (4a) is securely connected to the guide element (2) and wherein the shield (4) has a first and a second state and wherein the shield (4) in the first state has a conical shape and in the second state is deformed compared with this conical shape, **characterized in that** the guide element (2) has a first securing means (5, 5', 5") and the dilator (3) has a corresponding second securing means (6, 6', 6"), whereby the guide element (2) can be detachably connected by positive locking to the dilator (3) in a position in which a distal end of the dilator (3) is engaged with an inner surface of the shield (4b) when the latter is in the first state.

2. Device (1) according to claim 1, **characterized in that**, at its base (4c), the shield (4) in the first state has an outside diameter which is larger than the inside diameter of the tracheostomy tube (10) to be inserted with the device (1) at its distal end (11a), and preferably corresponds to the outside diameter of a tracheostomy tube (10) to be inserted with the device (1) at its distal end (11a), and in its second state has a smaller outside diameter than the inside diameter of the tracheostomy tube (10) to be inserted with the device (1) due to deformation.

3. Device (1) according to one of claims 1 or 2, **characterized in that** the second securing means (6, 6', 6") is located on the dilator (3) at the distal end (3a) and the first securing means (5, 5', 5") is located on the guide element (2) in an area which, in the unconnected state of the securing means (5, 5', 5", 6, 6', 6"), in the first state of the shield (4) is covered by the shield (4) and in the second state of the shield (4) is at least partially visible.

4. Device (1) according to one of the preceding claims, **characterized in that** the first securing means (5, 5', 5") and the second securing means (6, 6', 6") form a turn-lock fastener, preferably a threaded fastener (5, 6) or a bayonet fastener (5', 6').

5. Device (1) according to one of claims 1 to 3, **characterized in that** the first securing means (5, 5', 5") and the second securing means (6, 6', 6") form a detent connection (5", 6").

6. Device (1) according to one of the preceding claims, **characterized in that** the shield (4) is made of silicone or comprises silicone wherein the shield (4) has a grooved structure on its inner surface and the dilator (3) has a corresponding grooved structure at the distal end (3a), wherein the grooves of the grooved structure preferably extend in the longitudinal direction.

7. Device (1) according to one of the preceding claims, **characterized in that** the shield (4) with its inner surface (4b) lies against the distal end (3a) of the dilator (3) in the connected state of the securing means (5, 5', 5", 6, 6', 6") and in the first state of the shield (4) with an elastic pretension.

8. Set with a tracheostomy tube (10) and a device (1) according to one of claims 1 to 7.

9. Set according to claim 8, **characterized in that** the tracheostomy tube (10) has a cannula tube (11) with a proximal end (11b) and the dilator (3) in the area of its proximal end has a third securing means (7, 7'), with which the dilator (3) can be detachably connected to the proximal end (11b) of the cannula tube (11) and/or to a fourth securing means (12, 12') at the proximal end (11b) of the cannula tube (11).

10. Set according to claim 9, **characterized in that** the third securing means (12, 12') comprises a union nut or a clamp or a connector.

## Revendications

1. Dispositif (1) destiné à l'introduction d'une canule de trachéotomie, comprenant un élément de guidage (2) et un dilatateur (3), le dilatateur (3) étant de forme tubulaire et l'élément de guidage (2) comprenant un écran (4) avec une pointe (4a) et une paroi d'écran et une base (4c), l'élément de guidage passant par l'axe de l'écran (4) et la pointe (4a), et l'écran (4) étant solidaire de l'élément de guidage (2) dans la zone de la pointe (4a), et l'écran (4) présentant un premier et un deuxième état et l'écran (4) présentant, en le premier état, une forme conique et, en le deuxième état, étant déformé par rapport à cette forme conique, **caractérisé en ce que**
l'élément de guidage (2) comprend un premier moyen de fixation (5, 5', 5") et le dilatateur (3) comprend un deuxième moyen de fixation (6, 6', 6") correspondant, ce par quoi l'élément de guidage (2) est adapté pour être attaché au dilatateur (3), de manière amovible, par complémentarité de forme, dans une position dans laquelle une extrémité distale du dilatateur (3) est engagée dans une surface intérieure (4b) de l'écran lorsque celui-ci est en le premier état.

2. Dispositif (1) selon la revendication 1, **caractérisé en ce que** l'écran (4) présente à sa base (4c) dans le premier état un diamètre extérieur qui est plus grand que le diamètre intérieur de la canule de trachéotomie (10) à introduire avec le dispositif (1), à son extrémité distale (11a), correspond de préférence au diamètre extérieur d'une canule de trachéotomie (10) à introduire avec le dispositif (1), à son extrémité distale (11a), et dans son deuxième état par déformation présente un diamètre extérieur plus petit que le diamètre intérieur de la canule de trachéotomie (10) à introduire avec le dispositif (1).

3. Dispositif (1) selon une des revendications 1 ou 2, **caractérisé en ce que** le deuxième moyen de fixation (6, 6', 6") se trouve au dilatateur (3) à l'extrémité distale (3a) et que le premier moyen de fixation (5, 5', 5") se trouve à l'élément de guidage (2) dans une zone qui, dans un état non attaché des moyens de fixation (5, 5' 5", 6, 6' 6"), est recouvert par l'écran (4) dans le premier état de l'écran (4) et est visible, du moins partiellement, dans le deuxième état de l'écran (4).

4. Dispositif (1) selon l'une des revendications précédentes, **caractérisé en ce que** le premier moyen de fixation (5, 5', 5") et le deuxième moyen de fixation (6, 6', 6") forment un raccord rotatif, de préférence un raccord fileté (5, 6) ou un raccord à baïonnette (5', 6').

5. Dispositif (1) selon l'une des revendications 1 à 3, **caractérisé en ce que** le premier moyen de fixation (5, 5', 5") et le deuxième moyen de fixation (6, 6', 6") forment un raccord à cran (5", 6").

6. Dispositif (1) selon l'une des revendications précédentes, **caractérisé en ce que** l'écran (4) est fabriqué en silicone ou contient du silicone, l'écran (4) comprenant une structure striée à sa surface interne et le dilatateur (3) une structure striée correspondante à une extrémité distale (3a), le stries de la structure striée s'étendant de préférence dans le sens longitudinal.

7. Dispositif (1) selon une des revendications précédentes, **caractérisé en ce que**, dans l'état attaché des moyens de fixation (5, 5' 5", 6, 6', 6") et dans le premier état de l'écran (4), l'écran (4) est en appui, avec une précontrainte élastique, par sa surface interne (4b), sur l'extrémité distale (3a) du dilatateur (3).

8. Ensemble avec une canule de trachéotomie (10) et un dispositif (1) selon une des revendications 1 à 7.

9. Ensemble selon la revendication 8, **caractérisé en ce que** la canule de trachéotomie (10) comprend un tuyau de canule (11) avec une extrémité proximale (11b) et le dilatateur (3) comprend, dans la zone de son extrémité proximale, un troisième moyen de fixation (7, 7') par lequel le dilatateur (3) peut être attaché de manière amovible à l'extrémité proximale (11b) du tuyau de canule (11) et/ou à un quatrième moyen de fixation (12, 12') à l'extrémité proximale (11b) du tuyau de canule (11).

10. Ensemble selon la revendication 9, **caractérisé en ce que** le troisième moyen de fixation (7, 7') comprend un écrou-raccord ou une pince ou un connecteur.
